# EUROPEAN PATENT APPLICATION

(11) **EP 1 674 128 A1**
(43) Date of publication of application: **28.06.2006**
(21) Application number: 04106870.1
(22) Date of filing: 22.12.2004
(51) Int. Cl.: A61M 35/00

(54) **Magnetic pole matrices useful for tissue engineering and treatment of disease**

(71) Applicant: Steinbeis-Transferzentrum für Herz-Kreislaufforschung, 18057 Rostock (DE)
(72) Inventor: Wenzhong, Li, Dr., 18057, Rostock (DE); Steinhoff, Gustav, Prof. Dr.med., 18211, Rethwisch (DE); Nan, Ma, Dr., 18057, Rostock (DE); Steinhoff, Kurt, Prof. Dr.-Ing., 47533, Kleve (DE)
(74) Representative: Garrels, Sabine

(57) **Abstract**

A method relates to magnetic pole matrices suitable for tissue engineering and targeting systematic therapy for cardiovascular disease using magnetic polymer nanopartides gene/drug (various cytokines/growth factors/synthetic chemicals) delivery.

The magnetic pole matrices used in the present invention possesses advantages, such as distributing the magnetic nanoparticles conjugated with gene/drug (various cytokines/growth factors/synthetic chemicals) locally and uniformly on the artificial surface regulated by self-organising behavior benefited from the magnetic pole matrices, which essentially solved the blood vessel blocking problem related to systematic therapy by magnetic nanoparticles gene/drug delivery for cardiovascular disease; promoting the adhesion of the cells (stem cells/epithelial cells/endothelial cells) labeled with magnetic beads on specific location of the artificial surface, which is very important for tissue engineering.

## Description

### Technical field

A method relates to magnetic pole matrices suitable for tissue engineering and systematic therapy for cardiovascular disease using magnetic polymer nanoparticles gene/drug (various cytokines/growth factors/synthetic chemicals) delivery.

### Background art

**Systemtic therapy for cardiovascular disease**

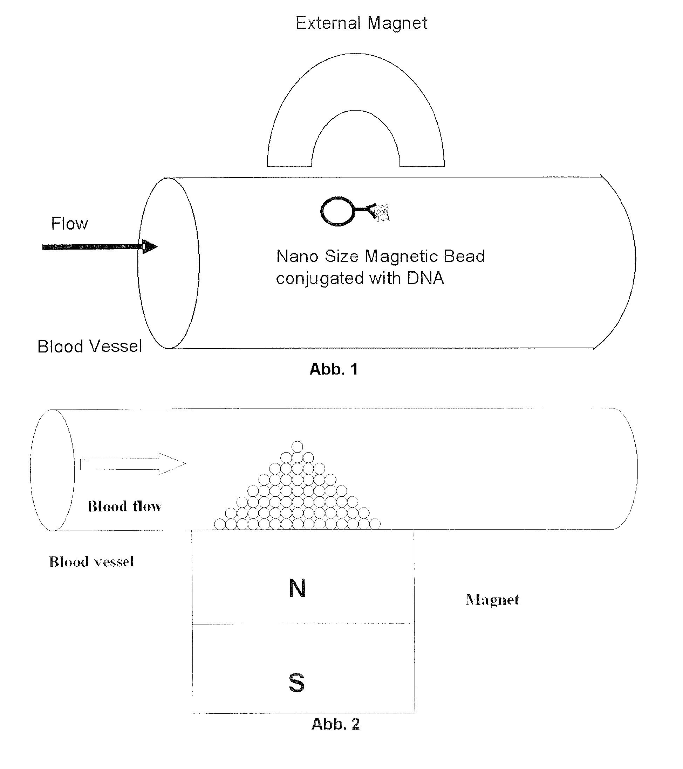

Targeting a specific area of the body is one of the main concerns associated with drug administration. Usually, large doses of the drug have to be administered to reach an acceptable therapeutic level at the desired site because only a fraction of the dose can actually reach the disired site. Further, the high dosages may also cause toxic side effects on the non-target organs (V.P. Torchilin, Drug targeting. Eur. J. Pharm. Sci. 11 Suppl. 2 (2000), pp. S81-S91). Hence targeting drug delivery to the desired site would reduce the quantity of drug required to reach local therapeutic levels at the target site, decrease the concentration of the drug at non-target sites and consequently reducing the possible side effects. Magnetic targeting drug administration incorporates magnetic particles into drug carriers, uses an externally applied magnetic field to physically direct these magnetic drug carrier particles to a desired site (S. Goodwin, C. Peterson, C. Hoh and C. Bittner, Targeting and retention of magnetic targeted carriers (MTCs) enhancing intra-arterial chemotherapy. J. Magn. Magn. Mater. 194 (1999), pp. 132-139)(S. Rudge, C. Peterson, C. Vessely, J. Koda, S. Stevens and L. Catterall, Adsorption and desorption of chemotherapeutic drugs from a magnetically targeted carrier (MTC). J. Control Release 74 (2001), pp. 335-340) as illustrated in Figure 1. The magnetic particles can be injected into the bloodstream and guided to the targeted area with external magnetic fields (S. Rudge, C. Peterson, C. Vessely, J. Koda, S. Stevens, and L. Catterall, "Adsorption and desorption of chemotherapeutic drugs from a magnetically targeted carrier (MTC)'" J. Controll. Rel., vol. 74, pp. 335-340, 2001)(G. A. Flores, "In-vitro blockage of a simulated vascular system using magnetorheological fluids as a cancer therapy'" Eur. Cells Mater., vol. 3, pp. 9-11, 2002). Further, the magnetic particles in the magnetic fluid can interact strongly with each other, which facilitate the delivery of high concentrations of drug to disred areas. Moreover, magnetic particles composed of magnetite are well tolerated by the human body. Also, magnetic fields are not screened by biological fluids and do not interfere with most biological processes, hence they are well suited for biological applications. However, there are still several problems associated with magnetic targeting in humans which limits its application. The first limitation is associated with the influence of blood flow rate at the target site on the accumulation of magnetic particles. Therefore, much stronger magnetic fields would be required to retain magnetic particles in large arteries. Another problem associated with magnetic drug targeting in humans is the depth of the target site. Sites that are more than 2 cm deep in the body are difficult to target because the strength of the magnetic field decreases with distance (S.R. Rudge, T.L. Kurtz, C.R. Vessely, L.G. Catterall and D.L. Williamson, Preparation, characterization, and performance of magnetic iron-carbon composite microparticles for chemotherapy Biomaterials 21 (2000), pp. 1411-1420). Moreover, although the strong interaction with each other magnetic nanoparticles may facilitate the delivery of high concentrations of drug to targeted areas, it may also aggregate into a blot, hence blocking the blood flowing in the vessel (H. Schewe, M. Takayasu, and F. J. Friendlaender, "Observation of particle trajectories in an HGMS single-wire system," IEEE Trans. Magn., vol. MAG-16, pp. 149-454, Jan. 1980)(F. J. Friedlaender, R. Gerber,W. Kurzl, and R. R. Birss, "Particle motion near and capture on single spheres in HGMS," IEEE Trans. Magn., vol. MAG-17, pp. 2801-2803, Nov. 1981)(F. J. Friedlaender, R. Gerber, H. P. Henkel, and R. R. Birss, "Particle buildup on single spheres in HGMS," IEEE Trans. Magn., vol. MAG-17, pp. 2804-2806, Nov. 1981) as shown in Figure 2.

**Tissue engineering**

Endothelial seeding on biomedical devices such as artifical heart valve, stent and vessel bypass grafts plays a important role in overcoming the risk of acute thrombosis and chronic instability of the implant surface (M. Reyes, T. Lund, T. Lenvik, D. Aguiar, L. Koodie and C.M. Verfaillie, Purification and ex vivo expansion of postnatal human marrow mesodermal progenitor cells. Blood 98 9 (2001), pp. 2615-3625)(S. Kaushal, G.E. Amiel, K.J. Guleserian et al., Functional small-diameter neovessels created using endothelial progenitor cells expanded ex vivo. Nat Med 7 9 (2001), pp. 1035-4040). The aims of this surface modification technique are to produce a confluent and biologically active surface with viable endothelial cells. Substantial efforts have been paid for in vitro engineering of endothelialized implants (E.L. Dvorin, J. Wylie-Sears, S. Kaushal, D.P. Martin and J. Bischoff, Quantitative evaluation of endothelial progenitors and cardiac valve endothelial cells: proliferation and differentiation on poly-glycolic acid/poly-4-hydroxybutyrate scaffold in response to vascular endothelial growth factor and transforming growth factor beta1. Tissue Eng 9 3 (2003), pp. 487-493)(Y. Zhao, D. Glesne and E. Huberman, A human peripheral blood monocyte-derived subset acts as pluripotent stem cells. Proc Natl Acad Sci USA 100 5 (2003), pp. 2426-2431). However, the lengthy preparation time to harvest, expand and culture the patient,s autologous cells, and the possible cell culture contamination greatly limit the application of in vitro endothelial seeding for biomedical devices. In vivo endothelial seeding through the recruitment of circulating magnetically modified target cells to the surface of biomedical devices capable of forming a magnetic interaction with target cells could effectively solve the problems associated with the in vitro endothelial seeding (Patent WO 03/037400). However, the magnetic field from the surface of devices may also cause the magnetically modified target cells aggregate into a blot on the surface of the device, which would form thrombosis in the implant surface and this may even block the blood flowing in the vessel as shown in Figure 2. Consequently, the normal physiological function of organs dependent on those vessels may be disturbed, or even cause the failure of the organs.

### Disclosure of the invention

### Technical problem

The problem of the invention is to provide an economical and effective method to solve above mentioned problem by magnetic pole matrices. It have to allow magnetic polymer nanoparticles gene/drug (various cytokines/growth factors/synthetic chemicals) delivery and the in vivo enothelial seeding for the biomedical device being utilized in tissue engineering and systematic therapy for cardiovascular disease.

### Technical solution

The solution of the Problem is a Magnetic pole matrix chip and a method of magnetic pole matrix therapy comprising:
(a) a substrate;
(b) micro-electromagnetic units on the substrate, which produce a magnetic field upon imposing electric current thereto;
(c) a first layer of conductive traces, each of traces extending at least 90° around one of the ferromagnetic cores;
(d) a second layer of conductive traces, each of traces extending at least 90° around one of the ferromagnetic cores, and being separated from the first set of conductive traces by an insulating layer penetrated by a verticle conductive connection between traces of said first layer and traces of said second layer;
(e) a functional layer for immobilizing ligand moelecules;
(f) ligand molecules immobilized by the functional layer;
(g) modifying target molecules to make the target molecules positionable by magnetic fields;
(h) modifying target cells to make the target cells positionable by magnetic fields;
(i) modifying ligand molecules to make the ligand molecules positionable by magnetic fields;
(j) selectively energizing magnetic cores to form desired local magnetic fields;
(k) magnetically guiding with a non-alternating magnetic field the magnetic component particle to a targeted site;

### Advantageous effects

The invention provides an effective method for local targeting magnetic polymer nanoparticles for gene/drug (various cytokines/growth factors/synthetic chemicals) delivery with wanted magnetic and biological properties in relation to tissue engineering and systematic therapy for cardiovascular disease using magnetic pole matrices.

In one aspect, the invention therefore provides a process of using magnetic pole matrices as tools to manipulate the magnetic nanoparticles for gene/drug delivery. It provides a more flexible local targeting strategy used in magnetic nanoparticles for gene/drug delivery. Employment of the magnetic pole matrices in the invention has several advantages, including providing a source of strong localized magnetic field gradients at defined locations in the body for targeted drug delivery, distributing the magnetic nanoparticles on the artificial surface locally and uniformly due to the self-organising behavior regulated by magnetic pole matrices, effectively solving the problem related to blood flow blocking due to the aggregation of magnetic nanoparticles in the vicinity of external magnet. The magnetic pole matrices suitable for the present invention is easily available and relatively inexpensive benefited from the large scale fabrication ability of mature VLSI, ULSI and MEMS (Micro-Electro-Mechanical Systems) technology. The magnetic pole matrices can be used not only in systematic therapy in cardiovascular diseases, but also in tissue engineering, such as growing cells on artificial surface in vitro and in vivo.

In other aspects the invention provides methods of controlling the drug/gene dosage administered by the magnetic nanoparticles. On the one hand, the aggregation of magnetic nanoparticles in the vicinity of external block magnet may cause over high dosages delivery, which can cause toxic side effects at the target organs. On the other hand, because it is difficult for an external magnet to produce a strong and localized magnetic field, external block magnet may trap almost all of the magnetic nanoparticles in a non-target or part of target area, hence limiting its application in directing magnetic nanoparticles to delivery drug/gene to the desired area. Use of magnetic pole matrices in the process of delivering can apparently reduce the non-target aggregation of the magnetic nanoparticles and increase the ability to manipulate the drug/gene delivered uniformly, hence control the drug/gene dosage delivered by the magnetic nanoparticles.

### Brief description of the drawings

Other novel aspects, features and advantages of the invention will become apparent to those of ordinary skill in the art up review of the following description of specific embodiments of the invention in conjunction with the accompanying figures.

In the figures, which illustrate exemplary embodiments of the invention,

Figure 1 is a model for trapping of circulating magnetic bead-Polymer-DNA complex by external magnet.

Figure 2 shows Circulating magnetic beads trapped by external magnet forming block in the blood vessel.

Figure 3 shows principle for magnetic pole matrix.

Figure 4 shows magnetic pole matrix uniformly distributes the circulating magnetic beads.

Figure 5 is scanning electron micrograph of magnetic pole matrices with pillars heights of 100nm and widths of 50nm

Figure 6 shows PEI-magentic beads transfection efficiency to HEK293 checked by luciferase.

Figure 7 shows PEI-magentic beads transfection efficiency to NIH3T3 checked by luciferase.

Figure 8 shows PEI-magentic beads transfection efficiency to COS7 checked by luciferase.

Figure 9 shows PEI-magentic beads transfection efficiency to TP67 checked by luciferase.

Figure 10 shows gene delivery to HEK-293 (200x) by magnetic nano-particles.

Figure 11 shows transfection specificity of magnetically controlled gene delivery.

Figure 12 shows magnetic beads tracking of magnetically controlled gene delivery.

### Mode(s) for carrying out the invention

The present invention provides an easy and effective process for uniformly distributing magnetic nanoparticles using magnetic pole matrices. The patterned magnetic pole matrices with feature size in nano range exhibit wanted magnetic properties for magnetic polymer nanoparticles gene/drug (various cytokines/growth factors/synthetic chemicals) delivery being utilized in tissue engineering and systematic therapy for cardiovascular disease. The magnetic pole matrix of the present invention can be advantageously performed on systematic therapy for cardiovascular disease and on endothelial seeding for biomedical devices so that the blocking caused by the aggregation of magnetic nanoparticles or the magnetically attracted targeting cells can be effectively eliminated.

**Fabriaction of magnetic pole matrices**

The magnetic pole matrix can be prepared by any suitable method known to a person skilled in the art and preferably by the MEMS and IT related technology with the potential for a large scale manufacture.

Electron-beam lithography enables fabrication of nano structures as small as 15 nm wide magnetic bars. X-ray lithography, imprint lithography and interferometric lithography are also available to pattern larger area samples with deep submicron feature size. Interferometric lithography could be applied to make square, rectangular, or oblique periodic arrays of circular or elongated particles, and it can cover areas of 10 cm diameter or greater in a rapid, economical process that does not require a mask. Self-assembled lithography methods, such as the use of anodized alumina or block-copolymer templates, also can be used to nanopattern large areas. Magnetic arrays were made using additive or subtractive processes, which is typical in MEMS and IT related technology. In this invention, additive processes include the deposition of magnetic material into a template, using either electrodeposition or evaporation and liftoff. In a subtractive process, the magnetic film or multilayer is deposited first then etched using wet or dry etching methods. Aperiodic features such as servo patterns to assist dynamic control of the magnetic fields and the bond pads for electrical connections, can be superposed using an additional lithography step.

**Application of magnetic pole matrices in systemtic therapy for cardiovascular disease**

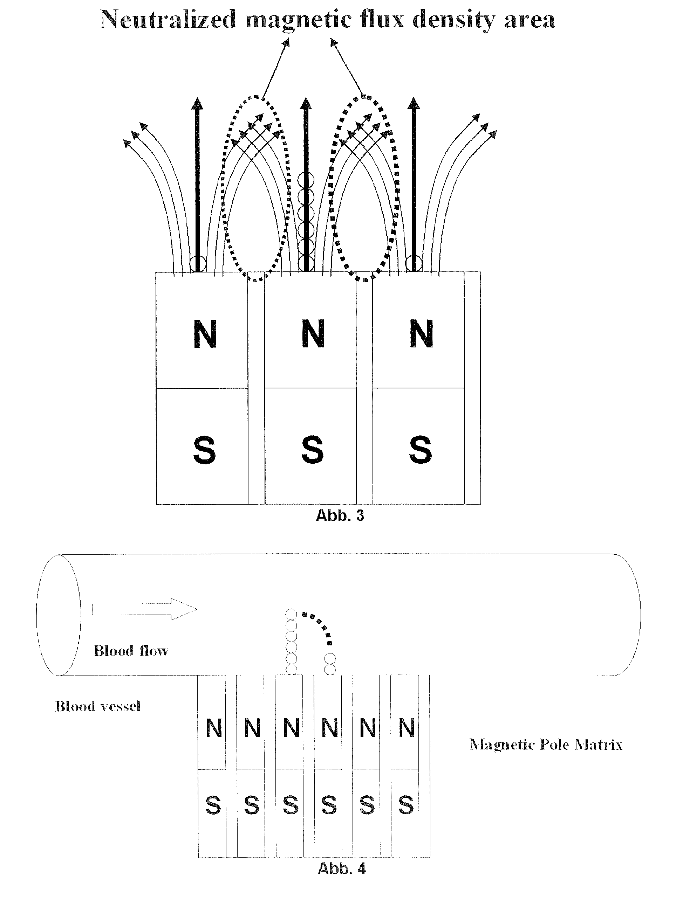

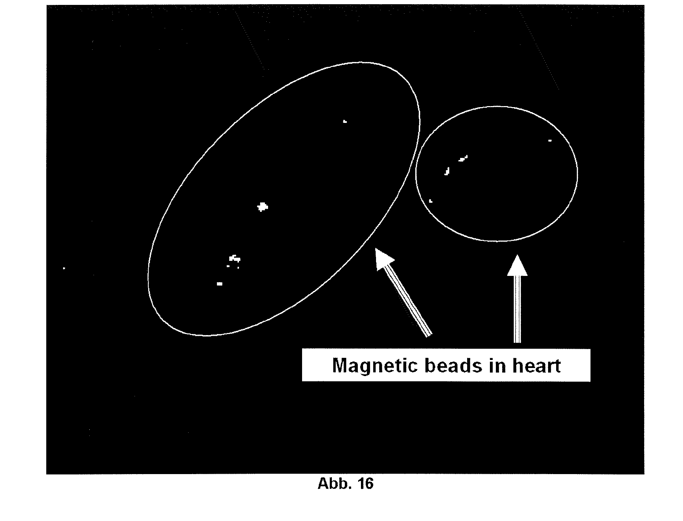

Although magnetic targeting drug administration shares many advantages over other delivery methods, the magnetic nanoparticles may also aggregate into blots, blocking the blood flow in the vessels shown as Figure 16. Consequently, the normal physiological function of organs dependent on those vessels may be disturbed, or even cause the failure of the organs. To effectively solve the problems associated with the magnetic particles aggregation, the magnetic pole matrix was employed in this invention. The principle of magnetic pole matrix was illustrated in Figure 3. As we know, magnetic field is the space around the magnet where its magnetic power or influence can be detected. The magnetic field is filled with magnetic lines of force. Magnetic line of force is the closed continuous curve in a magnetic field along which the north pole will move if free to do so, and its direction is given by the direction in which the isolated north pole will point.

Magnetic lines of force have the following main characteristic features.
► They are closed continuous curves.
► They never intersect each other.
► They mutually repel each other.
► They contract laterally, i.e., they bend along the length of the magnet.
► Outside the magnet, they travel from north to south.
► Inside the magnet, they travel from south to north.

Based on the characteristics of the magnetic lines of force, we arranged the magnetic poles in regular, repetitive pattern with equal distances between neighboring units to form the magnetic pole matrix. Hence, between each two poles, there forms a neutralized magnetic flux density area. When a magnetic particle falls to a location between two magnetic poles, the magnetic particle will be attracted to either the pole in its left side or the other pole in its right side as the position between two poles is not a stable balance position of magnetic particles. Further, each magnetic pole can not attract the magnetic particles without limitation, as with the accumulation of magnetic particles in the pole direction, the magnetic particle on the top position is in a non-stable balance position and a small disturb can make it drop to another position until it goes to a stable balance position as shown in Figure 4. In this way, the magnetic pole matrix can automatically distribute the magnetic particles uniformly on the top area of magnetic pole matrix. Thus, the magnetic pole matrix chips integrated with electromagnetic coils would not only provide a strong local magnetic field near the targeting organ but also distribute the magnetic particles uniformly in the desired zone. And more importantly, it effectively solves the problems associated with the aggregation of the magnetic particles and provides a controllable way for the magnetic targeting systematic drug administration.

**Application of magnetic pole matrices in tissue engineering**

Although in vivo magnetic endothelial seeding owns its unique potentials over other endothelial seeding methods, its application was greatly reduced due to the aggregation of magnetically modified target cells on the surface of the device, which forms the thrombosis in the implant surface. This may even block the blood flowing in the vessel. As a consequence, the normal physiological function of organs dependent on those vessels may be disturbed, or even cause the failure of the organs. Similarly, the magnetic pole matrix in this invention can also be employed to solve above mentioned problems associated with the aggregation of magnetically modified target cells on the surface of the device. Also the magnetic pole matrix chips integrated with electromagnetic coils will also provide a strong local magnetic field under the implants, enhancing the adhesion of targeting cells modified with magnetic particles to the surface of implants. And more importantly, it effectively solves the problems associated with the aggregation of the targeting cells modified with magnetic particles and provides a controllable way for magnetic enhanced in vivo and in vitro endothelial seeding on the surface of the medical implants.

The following examples with reference to the accompanying drawing illustrate the present invention but are not limiting as to the nature of the invention.

**EXAMPLE 1 - Magnetic pole matrices fabrication**

A magnetic pole matrix was formed as follows:

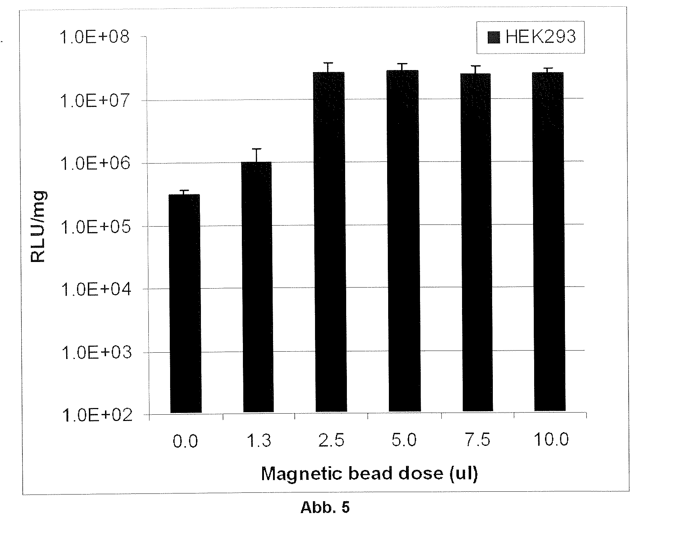

In this example, electron beam lithography and electroplating were used to produce nanoscale pillar arrays. A plating base of 10 nm Ti and 20 nm Au were evaporated on a silicon substrate. The substrate is then spin coated with polymethyl methacrylate (PMMA) positive resist of 950kD in molecular weight. The final thickness of the PMMA was 200 nm, which determined the maximum height of the pillars. The arrays of small holes were exposed and developed in PMMA resist using electron beam lithography. The resulting structure was used as a template for the sputtering deposition of magnetic pillars. Next, magnetic arrays were made using sputtering and liftoff to deposit magnetic material into the template. In the sputtering process, magnetic film is formed over the photoresist mask. As a result, the thickness of pole matrix layer thus obtained can be determined by the sputtering rate and the sputtering time. Aperiodic features such as servo patterns to assist dynamic control of the magnetic fields and the bond pads for electrical connections, can be superposed using an additional lithography step. After the sputtering, the PMMA was removed in the acetone bath to leave the magnetic pillar arrays, shown in Figure 5.

**EXAMPLE 2 - Gene delivery in vitro**

Gene therapy in cardiovascular system is mainly limited due to the low transfection efficiency of gene vectors in blood, in which the serum may degrade the vector,s ablity to delivery genes.

In this example, the non-viral gene vector poly-ethyleneimine (PEI) was covalently conjugated with magnetic nanobeads and desired gene by Sulfo-NHS-LC-Biotin linker to evaluate the transfection efficiency improvement. The magnetic beads/PEI/DNA complexes were found very stable even in medium with serum.

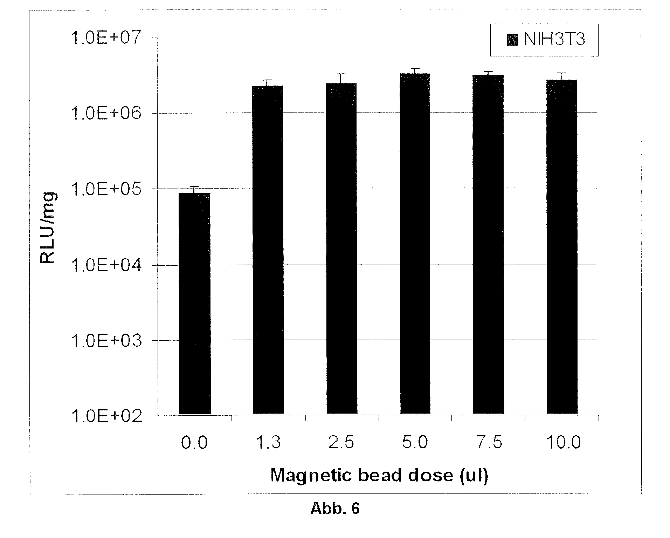

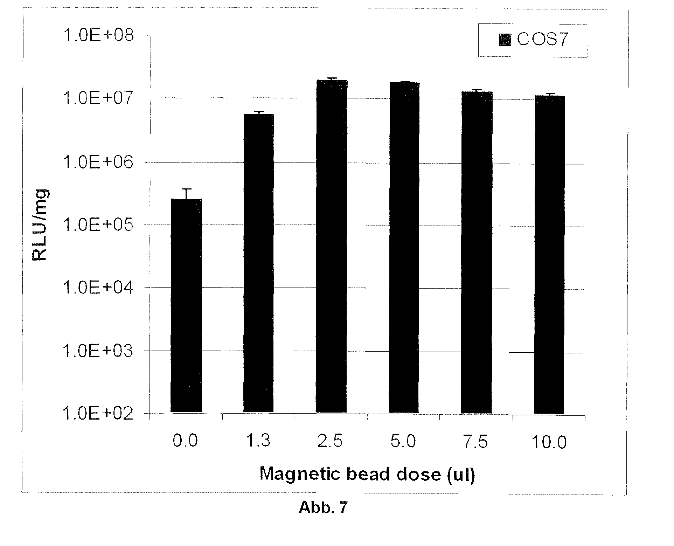

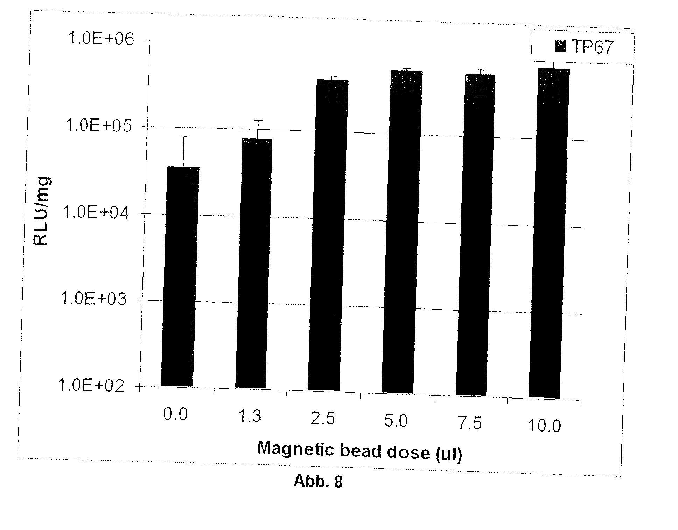

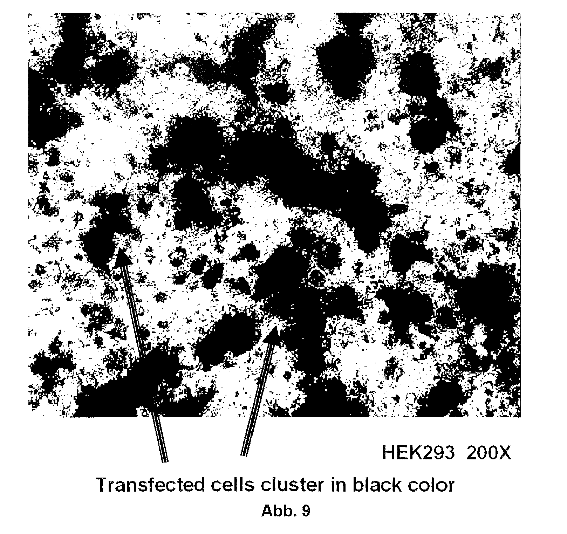

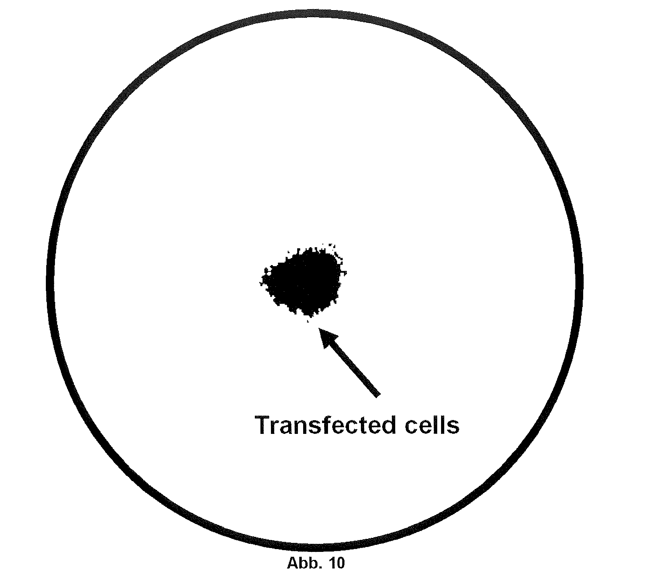

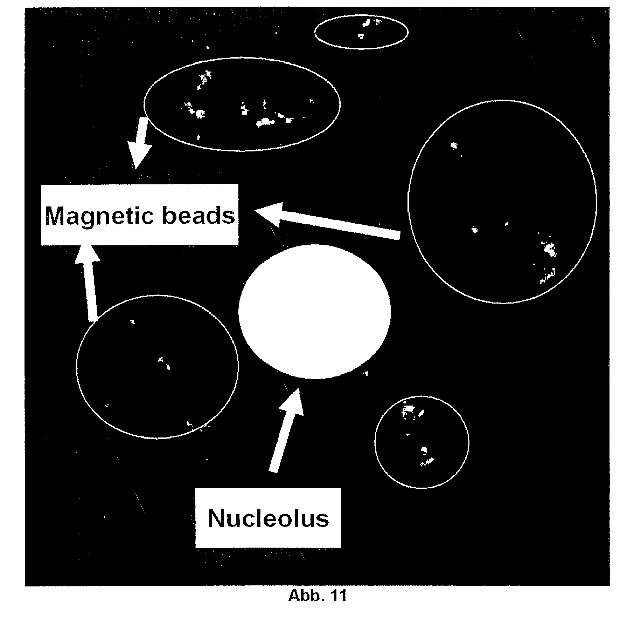

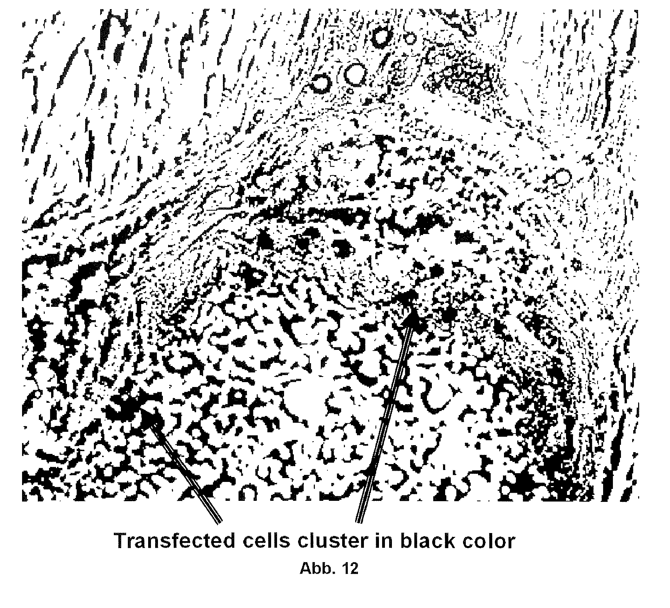

It was found that magnetic beads/PEI/DNA complexes prepared in medium with serum has about 100 fold increasment of transfection efficiency than PEI/DNA complexes in 4 different cell lines tested by luciferase reporter gene as shown in Figure 6, Figure 7, Figure 8 and Figure 9. By applying a restricted external magnetic field to 2D cell cultures, LacZ gene transfection (shown in Figure 10) could be selectively targeted to a specific and localized cell population as illustrated in Figure 11. By using confocal microscopy to track the magnetic bead/PEI/DNA complex, effective vector endocytosis and extranuclear localization in lysosomes was demonstrated shown as in Figure 12. Magnetic nanobeads conjugated with non-viral polymer vector provide superior transfection efficiency in vitro and in myocardium in vivo, which can be locally focussed by external magnetic fields. Circumventing virus associated problems, this technique can greatly enhance the prospects of gene therapy in the cardiovascular system.

**EXAMPLE 3 - Gene delivery in vivo**

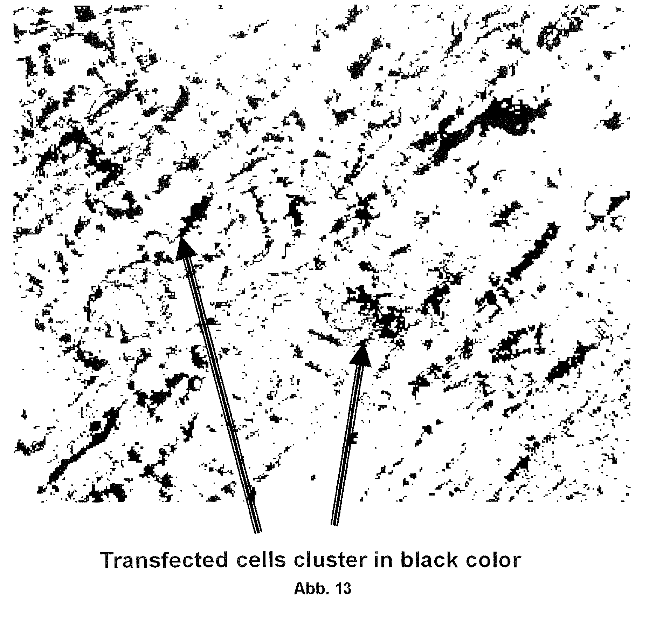

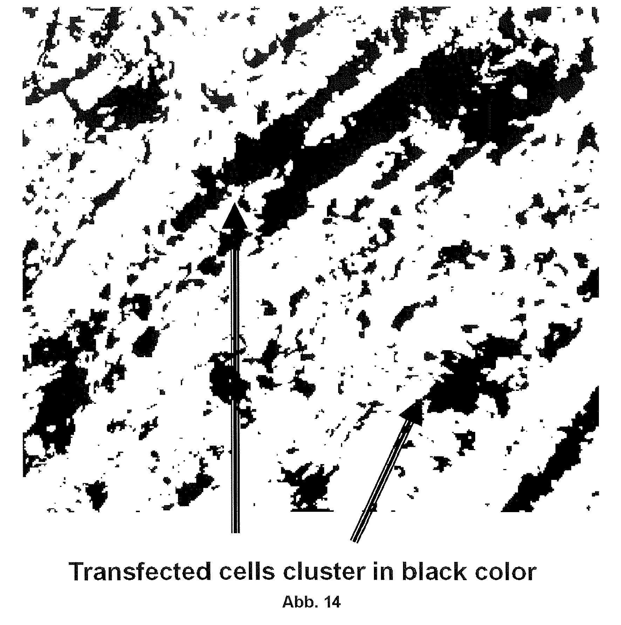

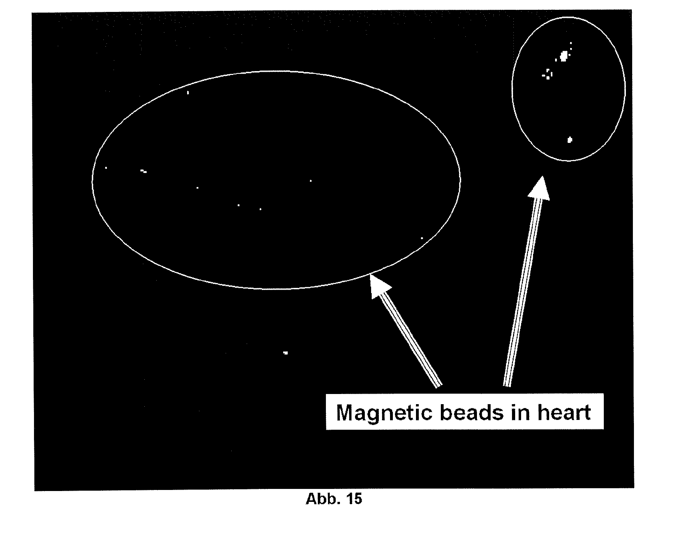

In this example, the non-viral gene vector poly-ethyleneimine (PEI) was covalently conjugated with magnetic nanobeads and reporter gene LacZ by Sulfo-NHS-LC-Biotin linker to evaluate the transfection efficacy in mouse mode. The magnetic beads/PEI/DNA complexes were prepared in medium with serum. The magnetic beads/PEI/DNA complexes with volume 50 µl were injected into the leg muscle of the mouse. LacZ gene expressions were found in the leg muscle after 72 hours injection as shown in Figure 13, Figure 14 and Figure 15. It is demonstrated that the present invention provides a feasible gene/drug delivery strategy for cardiovascular system disease.

**EXAMPLE 4 - Systematic therapy feasible in liver, brain, spleen, heart and kidney**

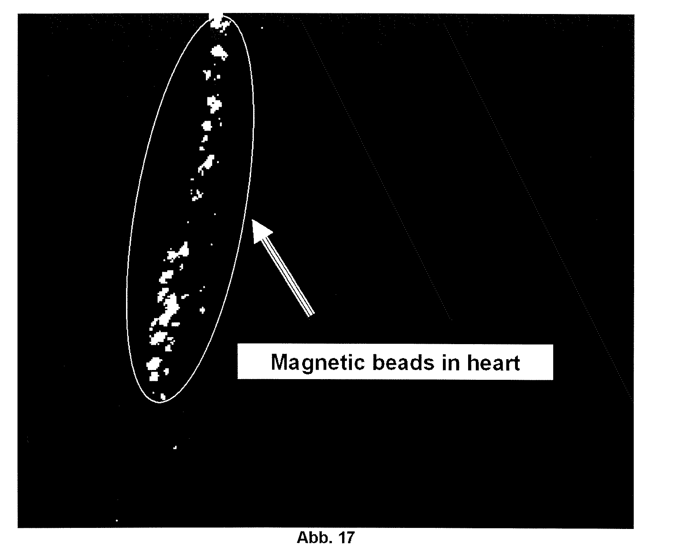

In this example, the non-viral gene vector poly-ethyleneimine (PEI) was covalently conjugated with magnetic nanobeads and fluorescent probe Oregon Green 488 by Sulfo-NHS-LC-Biotin linker to evaluate the feasiblity of systematic therapy. The magnetic beads/PEI/Florescent Probe complexes with volume 50 µl prepared in medium with serum entered blood circulation system of mouse by the tail vein injection. The external magnet was put on the chest of the mouse for 2 hours to attract the magnetic particles circulating in the blood system. The magnetic particles were found in the heart after 72 hours injection as shown in Figure 16, Figure 17 and Figure 18. It is demonstrated that the present invention provides feasible systematic therapy for cardiovascular system disease.

Other features, benefits and advantages of the present invention not expressly mentioned above can be understood form this description and the accompanying drawings by those skilled in the art.

The gene/drug delivery by magnetic nanoparticles manipulated by the magnetic pole matrix and the process for forming them described herein are all exemplary embodiments of one or more aspects of the invention. As can be understood by a person skilled in the art, many modifications to these exemplary embodiments are possible. The invention, rather, is intended to encompass all such modification within its scope, as defined by the claim.

All documents referred to herein are fully incorporated by reference.

Although the invention has been described with reference to particular embodiments, the description is only an example of the invention's application and should not be taken as a limitation. Various adaptations and combinations of features of the embodiments disclosed are within the scope of the invention as defined by the following claims.

## Claims

1. Magnetic pole matrix chip and a method of magnetic pole matrix therapy comprising:
(a) a substrate;
(b) micro-electromagnetic units on the substrate, which produce a magnetic field upon imposing electric current thereto;
(c) a first layer of conductive traces, each of traces extending at least 90° around one of the ferromagnetic cores;
(d) a second layer of conductive traces, each of traces extending at least 90° around one of the ferromagnetic cores, and being separated from the first set of conductive traces by an insulating layer penetrated by a verticle conductive connection between traces of said first layer and traces of said second layer;
(e) a functional layer for immobilizing ligand moelecules;
(f) ligand molecules immobilized by the functional layer;
(g) modifying target molecules to make the target molecules positionable by magnetic fields;
(h) modifying target cells to make the target cells positionable by magnetic fields;
(i) modifying ligand molecules to make the ligand molecules positionable by magnetic fields;
(j) selectively energizing magnetic cores to form desired local magnetic fields;
(k) magnetically guiding with a non-alternating magnetic field the magnetic component particle to a targeted site;

2. The magnetic pole matrix chip of Claim 1 wherein said the substrate is a material selected from the group of silicon, glass, ceramic, silicon dioxide, plastic, and mixtures thereof.

3. The magnetic pole matrix chip of Claim 1 wherein said each microelectromagnetic unit comprises one magnetic core on the substrate and means for supplying electric current to the traces around the magnetic core.

4. The magnetic pole matrix chip of Claim 1 wherein said each microelectromagnetic unit comprises more than one magnetic core on the substrate.

5. The magnetic pole matrix chip of Claim 1 wherein said means for supplying electric current to the traces around the magnetic core comprises single or multiple loops of electric conductive traces around the magentic core.

6. The magnetic pole matrix chip of Claim 5 wherein said the loops of electric conductive traces are of a square, a circular, a triangular, a sprial, or a shape combination thereof.

7. The magnetic pole matrix chip of Claim 5 wherein said means for supplying electirc current to the traces further comprising means for modulating a magnitude and a polarity of the electric current selectively applied to any one of the units.

8. The magnetic pole matrix chip of Claim 5 wherein said means for supplying electirc current to the traces further comprising means for modulating a magnitude and a polarity of the electric current selectively applied to more than one of the units.

9. The magnetic pole matrix chip of Claim 1 wherein said the magnetic core comprises a ferromagnetic material or a ferrimagnetic materials, or mixtures thereof.

10. The magnetic pole matrix chip of Claim 1 wherein said the microelectromagnetic units are arranged on the substrate in a regular, repetitive pattern with equal distances between neighboring units.

11. The magnetic pole matrix chip of Claim 1 wherein said the microelectromagnetic units have dimensions of width and length ranging from 10nm to 1 cm.

12. The magnetic pole matrix chip of Claim 1 wherein said methods for applying an electric current to any one or more of the units to produce a magnetic field comprises conductive connections between each microelectromagnetic unit and a source of electric current and switch means for establishing a desired electric current through the conductive connections.

13. The magnetic pole matrix chip of Claim 12 wherein said the switch means are either mechanical or electronic switches, or combination of thereof.

14. The magnetic pole matrix chip of Claim 1 wherein said insulating layer separates the first layer of conductive traces from the second layer of conductive traces.

15. The magnetic pole matrix chip of Claim 1 wherein said the ligand molecules are selected from the group consisting of oligonucleotides, DNA molecules, RNA molecules, proteins, antibodies, lectins, and receptor molecules.

16. The magnetic pole matrix chip of Claim 1 wherein said modifying ligand molecules comprises linking the ligand molecules to magnetic material.

17. The magnetic pole matrix chip of Claim 1 wherein said modifying target cells comprises linking the target cells to magnetic material.

18. The magnetic pole matrix chip of Claim 16 wherein said linking the ligand molecules /target cells to magnetic materials is achieved by a covalent bond or biological affinity.

19. The magnetic pole matrix chip of Claim 18 wherein said biological affinity is selected from the group consisting of antibody-antigen, lectin-hapten, and receptor-ligand affinity.

20. The magnetic pole matrix chip of Claim 1 wherein said modifying target molecules/cells comprises linking the target molecules/cells to magnetic materials.

21. The method of Claim 1, wherein the magnetic component particles comprises a magnetosorptive particle or/and magnetocarbon particles.

22. The method of Claim 21, wherein the magnetosorptive composition comprises magnetoceramic particles.

23. The method of Claim 22, wherein the ceramic is selected from the group consisting of a natural porous adsorptive material and a synthetic porous adsorptive material.

24. The method of Claim 22, wherein the ceramic is selected from the group consisting of a natural porous adsorptive material and a synthetic porous adsorptive material.

25. The method of Claim 22, wherein the magnetoceramic particles further comprises one or more biologically active agents.

26. The method of Claim 22, wherein the one or more biologically active agents are selected from the group consisting of antibiotics, antifungals and antineoplastic agents.

27. The method of Claim 1, wherein the magnetic component particles are magnetopolymer particles.

28. The method of Claim 27, wherein the polymeric components are biodegradable polymers.

29. The method of Claim 1, wherein one or more biologically active bifunctional agent are attached to the particles.

30. The method of Claim 1, wherein the size of the particles is ranging from 10nm to 1 cm.

31. The method of Claim 1, wherein the particles are introduced by a method selected from the group consisting of injection, infusion, implantation, injestion and mixtures of thereof.

32. The method of Claim 1, wherein the targeted site is selected from the group consisting of organs, implantation devices, tumors, infections, aneurysms, abscesses, viral growths, and other focal points of disease.

33. The method of Claim 32, wherein the implantation devices is selected from the group consisting metal, biocompatible, biodegradable, polymer, ceramics, biological, synthetic device and any combination of thereof.

34. The method of Claim 1, also comprising the introduction of an embolic agent.

35. The method of Claim 34, wherein the embolic agent is a second batch of magnetic component particles, wherein the larger particles are used as the embolic agent.

36. The targetable particle in Claim 1, further comprising one or more delivery vehicles.

37. The magnetic component particle in Claim 1, is such that the one or more biologically active agents can be associated with the particle by adsorbed, grafted, encapsulated, or linked to the particle.

38. The said 'associated with, in Claim 37, means that the biologically active agent can be physically encapsulated or entrapped with the particle, dispersed partially or fully through the particle, or attached or linked to the particle or any combination thereof.

39. The said 'attachment or linkage, in Claim 38 is by means of covalent bonding, hydrogen bonding, adsorption, adsorption chelation, metallic bonding, van der Walls force or ionic boinding, or any combination thereof.

40. The said association of the biologically active agent(s) and the magnetic component particle(s) in Claim 38, may optionally employ connectors and/or spacers to facilitate the preparation or use of the conjugates.

41. The suitable connecting groups in Claim 40, are groups which link a biologically active agent to the particle without signifcantly impairing the effectiveness of the biologically active agent or the effectiveness of any other carried material present in the particle, and they are typically used in order to avoid steric hindrance between the biologically active agent and the particle.

42. The targetable cells in Claim 1, include progenitor cells, red blood cells, mononuclear cells, macrophages, cells of immune system.

43. The said cells of immune system in Claim 42, include T helper cells, platelets and progenitor cells.

44. The progenitor cells, wherein said in Claim 42, comprise endothelial cells.

45. The targetable cells in Claim 1, comprise autologous cells and donor cells.

46. The targetable cells in Claim 1, may be harvested from bone marrow or fat tissue and /or cultured in vitro.

47. The target cells in Claim 1, comprise modifying treatment with VEGF, G-CSF, GM-CSF or by ischemia.

48. The target cells in Claim 1, comprise genetically manipulated target cells.

49. The target cells in Claim 1, wherein said modifying target cells is carried out in vivo, in vitro or mixtures of thereof.

50. The method of Claim 1, wherein said further comprising altering a surface characteristics of blood contacting surface by said target cells.

51. The method of Claim 50, wherein said altering of said blood contacting surface by said target cells facilitates the in vivo formation of a cellular tissue on said blood contacting surface.

52. The method of Claim 51, wherein said cellular tissue is a tissues selected from the group of endothelial, fibrous, epithelial and bone tissue.

## Amended claims

### Amended claims in accordance with Rule 86(2) EPC.

**1.** Magnetic pole matrix chip comprising:
(a) a substrate;
(b) a magnetic unit on the substrate, which produces a local magnetic field to magnetize the magnetic layer and the magnetic poles layer.
(c) a magnetic layer;
(d) a magnetic poles layer;
(e) a polymer layer;

**2.** The magnetic pole matrix chip of claim 1 wherein said the substrate material is silicon, and the substrate is coated by biocompatiable ceramic or polymer.

**3.** The magnetic pole matrix chip of claim 1 wherein said magnetic unit is electromagnetic unit or paramagnetic unit.

**4.** The magnetic pole matrix chip of claim 3 wherein said electromagnetic comprises one magnetic core and electric coils around the magnetic core, which is activated by supplying electric current to the electric coils around the magnetic core.

**5.** The magnetic pole matrix chip of claim 3 wherein said paramagnetic unit material is paramagnetic or superparamagnetic materials, which is activated by external magnetic equipment.

**6.** The magnetic pole matrix chip of claim 4 wherein said magnetic core material is soft magnetic materials.

**7.** The magnetic pole matrix chip of claim 5 wherein said external magnetic equipment comprises magnetic resonance imaging (MRI) equipment, or other equipments which can produce sufficiently strong magnetic field to magnetize paramagnetic unit.

**8.** The magnetic pole matrix chip of claim 1 wherein said magnetic layer and magnetic poles layer material comprises soft magnetic materials, paramagnetic materials, superparamagnetic materials, and mixtures thereof.

**9.** The magnetic pole matrix chip of claim 1 wherein said polymer layer material is biocompatible material.

**10.** The magnetic pole matrix chip of claim 1 wherein said magnetic poles have sizes of width and length ranging from 10nm to 1cm.

**11.** The magnetic pole matrix chip of claim 1 wherein said magnetic unit on the substrate comprises adjustment means for controlling the magnetic field magnitude of magnetic unit to establish a desired magnetic field magnitude on the surface of the magnetic pole matrix.

**12.** The magnetic pole matrix chip of claim 1 wherein said the magnetic poles are arranged in a regular, repetitive pattern with equal distances between neighboring units, to establish the neutralized magnetic flux density area between each two poles.
